# EUROPEAN PATENT SPECIFICATION

(11) **EP 1 734 035 B1**
(45) Date of publication and mention of the grant of the patent: **16.04.2008**
(21) Application number: 06011048.3
(22) Date of filing: 30.05.2006
(51) Int. Cl.: C07C 263/10, C07C 263/20, C07C 265/14

(54) **Process for the preparation of 4,4'-diphenylmethane diisocyanate**
Verfahren zur Herstellung von 4,4'-Diphenylmethan Diisocyanat
Procédé pour la préparation de 4,4'-diphenylmethane diisocyanate

(30) Priority: 10.06.2005 DE 102005026864
(43) Date of publication of application: 20.12.2006
(73) Proprietor: Bayer MaterialScience AG, 51368 Leverkusen (DE)
(72) Inventor: Keggenhoff, Berthold, 47839 Krefeld (DE); Echterhoff, Ralf, 41539 Dormagen (DE); Serra, Ricardo, 201107 Shanghai (CN)

(56) References cited:
- EP-A- 0 482 490
- EP-A- 1 475 367
- US-A- 5 258 417
- US-A1- 2004 171 869
- US-A1- 2005 020 797
- US-B1- 6 576 788

## Description

### BACKGROUND OF THE INVENTION

The present invention relates to a process for the preparation of 4,4'-diphenylmethane diisocyanate (4,4'-MDI) which may be conducted in stages at two different sites.

Conventionally, 4,4'-MDI is produced industrially by the acid-catalyzed condensation of aniline with formaldehyde, reaction of the resulting polyamine mixtures with phosgene to give a mixture of MDI isomers and homologues (diisocyanates and polyisocyanates of the diphenylmethane series) and subsequent distillative separation of the mixture to give technically pure 4,4'-MDI, polymeric MDI and optionally other isomer mixtures. This process is carried out in interconnected production plants on one production site. As described in EP-A-1475367, for example, the first step of this process is to prepare a mixture of MDI isomers and homologues (diisocyanates and polyisocyanates of the diphenylmethane series). A partial distillate of isomeric diphenylmethane diisocyanates containing 4,4'-MDI as the main constituent is then separated from this mixture, the bottom product being used industrially as polymeric MDI. Pure 4,4'-MDI is then separated from the other isomers and sundry by-products in this partial distillate.

It can be advantageous, however, to carry out these process steps on two production sites that optionally are very remote from one another. For such two site production, the process stages up to the mixture of MDI isomers and homologues (diisocyanates and polyisocyanates of the diphenylmethane series) could take place at a first production site and the subsequent, distillative separation of the mixture to give 4,4'-MDI, polymeric MDI and optionally other isomer mixtures could be carried out at a second production site. Such a procedure would be economically attractive if, e.g., favorable raw material and infrastructure conditions are available in one country, but important customer markets with an obligation to local production of the end product are available in another, very remote country. One particular advantage of producing 4,4'-MDI in the immediate vicinity of the customer markets arises from the fact that the product is solid at ambient temperature, but can only be stored for a limited time in the liquid state because of its tendency to dimerize. Consequently, shipping over long distances requires expensive transportation in the solid state with constant cooling. For the same reason, neither pure 4,4'-MDI nor the impure crude distillate used for its preparation is suitable for transportation in bulk form over longer periods. By ship, pure 4,4'-MDI is transported in barrels (vats, drums) in the solid state with cooling. The product must then normally be melted again before use. It is therefore readily apparent that transportation of large quantities of 4,4'-MDI over long distances is a very expensive process.

US-A-5,258,417 describes a process for the preparation of storable polyisocyanate mixtures containing 60 to 75 wt.% of 4,4'-MDI, 4 to 10 wt.% of 2,4'-MDI and less than 1 wt.% of 2,2'-MDI, the remainder being 3,4- and higher-nuclear MDI homologues (trifunctional and higher-functional polyisocyanates). These mixtures are prepared by mixing monomeric and polymeric MDI and are stable on storage at 25°C. The patent does not include any teaching with respect to the suitability of these mixtures for transportation by ship, for example, so that they can subsequently be used to prepare 4,4'-MDI on a very remote production site.

### BRIEF DESCRIPTION OF THE INVENTION

The object of the present invention is to provide a simple process for the preparation of 4,4'-MDI by the acid-catalyzed condensation of aniline with formaldehyde, reaction of the resulting polyamine mixtures with phosgene to give a mixture of MDI isomers and homologues, and a subsequent distillative separation of the mixture to give 4,4'-MDI, polymeric MDI and optionally other isomer mixtures at a different location. It is also an object of the present invention to provide a process in which a raw material suitable for the preparation of 4,4'-MDI, for example a suitable MDI mixture, can be transported from a first production site to a second production site without the disadvantages of prior art processes.

These and other objects which will be apparent to those skilled in the art are accomplished by acid-catalyzed condensation of aniline with formaldehyde, reaction of the resulting polyamine mixture(s) with phosgene to give a mixture of MDI isomers and homologues (diisocyanates and polyisocyanates of the diphenylmethane series) at a first production facility. At a second, optionally remote location, the mixture of MDI isomers and homologues is distilled to separate the mixture into fractions of 4,4'-MDI, polymeric MDI and optionally other isomer mixtures.

### DETAILED DESCRIPTION OF THE INVENTION

The present invention provides a process for the preparation of 4,4'-diphenylmethane diisocyanate in which
(a) aniline and formaldehyde are reacted in the presence of an acid catalyst to give diamines and polyamines of the diphenylmethane series, at a first production site and
(b) the diamines and polyamines are reacted with phosgene at the first production site to give the corresponding diisocyanates and polyisocyanates of the diphenylmethane series, which may optionally be separated by distillation to give a mixture of diisocyanates and polyisocyanates containing 50 to 80 wt.%, preferably 55 to 75 wt.% and most preferably 55 to 70 wt.% of 4,4'-diphenylmethane diisocyanate; 1 to 12 wt.%, preferably 4 to 10 wt.% and most preferably 5 to 9 wt.% of 2,4'- and/or 2,2'-diphenylmethane diisocyanate taken together; and 10 to 45 wt.%, preferably 20 to 40 wt.% and most preferably 30 to 40 wt.% of trifunctional and higher-functional polyisocyanates, based on the weight of the mixture of diisocyanates and polyisocyanates,
c) the mixture of diisocyanates and polyisocyanates is transferred to transport containers and transported to a second production site remote from the first, and
d) the mixture of diisocyanates and polyisocyanates is separated by distillation and/or crystallization to give a pure 4,4'-diphenylmethane diisocyanate containing at least 97 wt.% of 4,4'-diphenylmethane diisocyanate and a maximum of 3 wt.% of 2,4'-diphenylmethane diisocyanate, preferably at least 98 wt.% of 4,4'-diphenylmethane diisocyanate, a maximum of 2 wt.% of 2,4'-diphenylmethane diisocyanate and a maximum of 0.1 wt.% of 2,2'-diphenylmethane diisocyanate.

In a preferred embodiment of the invention, only a first fraction of 10 to 80 wt.%, preferably of 20 to 50 wt.%, of the total amount of diisocyanate and polyisocyanate prepared in step b) by phosgenation is transferred to transport containers and transported to the second production site, optionally after distillative separation or purification and back-mixing. The remaining fraction is processed further on the first production site or optionally another production site, or marketed.

Preferably the second production site is at least 10 km, more preferably at least 100 km and most preferably at least 1 000 km remote from the first production site. Preferably the transportation is performed by ship.

The industrial preparation of diamines and polyamines of the diphenylmethane series in step a) generally takes place in two stages. In the first stage, aniline is condensed with formaldehyde in the presence of an acid catalyst to produce the corresponding MDA mixture. The acid catalyst used is conventionally a strong mineral acid such as aqueous hydrochloric acid. The proportions of 4,4'-diphenylmethane diamine and its isomers and homologues can be controlled by choosing the proportions of aniline, formaldehyde and mineral acid and the temperature and residence time conditions. The condensation can be carried out industrially by either a continuous or a batch method. A large number of processes for the preparation of MDA by the acid-catalyzed condensation of aniline have been disclosed (See, e.g., WO-A-99/40059, WO-A-99/54289).

In step b) of the process of the present invention, the diamines and polyamines of the diphenylmethane series, optionally after neutralization and phase separation and possibly other purification steps, are then converted to the corresponding diisocyanates and polyisocyanates of the diphenylmethane series by reaction with phosgene. This reaction conventionally takes place in an inert solvent such as chlorobenzene, dichlorobenzene or toluene. Solutions of amine and phosgene are mixed and then reacted by heating. The hydrogen chloride simultaneously formed and excess phosgene are then separated off and the solvent used is separated off, normally in stages. After complete separation of the solvent, the diisocyanates and polyisocyanates of the diphenylmethane series are obtained as the bottom product. A large number of processes for the preparation of diisocyanates and polyisocyanates of the diphenylmethane series by reacting MDA with phosgene are also known (See, e.g., WO-A-99/54289).

In one embodiment of the present invention, the mixtures of diisocyanates and polyisocyanates transferred and transported in step c) which contain 50 to 80 wt.% of 4,4'-diphenylmethane diisocyanate, 1 to 12 wt.% of 2,4'- and 2,2'-diphenylmethane diisocyanate taken together, and 10 to 45 wt.% of trifunctional and higher-functional polyisocyanates, based on the weight of the mixture of diisocyanates and polyisocyanates, are obtained by choosing the proportions of aniline, formaldehyde and mineral acid and the temperature and residence time conditions in the preparation of MDA in step a) to give the desired proportions of 4,4'-MDI and the other isomers and homologues, so that the corresponding MDI mixture will be obtained directly after reaction with phosgene in step b). A molar ratio of aniline to formaldehyde ranging from 1.9:1 to 3:1 and a molar ratio of aniline to mineral acid (hydrochloric acid) ranging from 10:1 to 2:1 have proven to be suitable proportions in the preparation of MDA. The temperature and residence time conditions are typically chosen so that the initial acid-catalyzed reaction of aniline with formalin and mineral acid, or the reaction of a pre-condensate, obtained by the non-acid-catalyzed preliminary reaction of aniline and formalin, with mineral acid, is started at 35 to 50°C with a residence time of 10 to 30 min, after which the temperature of the reaction mixture is raised uniformly to a final temperature of 100 to 150°C over 60 to 200 min.

In another embodiment of the present invention, diamines and polyamines containing a lower proportion of 4,4'-MDA of 40 to 55 wt.%, based on the weight of the diamines and polyamines, are first produced in the condensation of aniline and formaldehyde in step a), and are then phosgenated to the corresponding diisocyanates and polyisocyanates. A partial distillate of, e.g., 5 to 25 wt.% is then produced from these crude diisocyanates and polyisocyanates by flash evaporation under vacuum. This partial distillate preferably has a composition of 85 to 95 wt.% of 4,4'-MDI, 5 to 15 wt.% of 2,4'-MDI and max. 1 wt.% of 2,2'-MDI. The bottom product obtained is preferably a commercially conventional MDI mixture containing a reduced proportion of diisocyanate (also called MDI polymer, e.g., the commercial product sold under the name Desmodur^{®} 44V20 which is available from Bayer MaterialScience AG). The bottom product preferably has a composition of 35 to 45 wt.% of 4,4'-MDI, 2 to 7 wt.% of 2,4'-MDI and less than 1 wt.% of 2,2'-MDI, together with 50 to 60 wt.% of trifunctional and higher-functional polyisocyanates.

Because the partial distillate is unsuitable for transportation over longer distances due to its high crystallization point of over 30°C and its tendency to form insoluble components, it is then mixed again, in a weight ratio preferably of 1:1 to 1:4 (partial distillate to MDI polymer), with MDI polymer having a preferred composition of 35 to 45 wt.% of 4,4'-MDI, 2 to 7 wt.% of 2,4'-MDI and less than 1 wt.% of 2,2'-MDI, together with 50 to 60 wt.% of trifunctional and higher-functional polyisocyanates, to give the transportable mixture of diisocyanates and polyisocyanates transferred in step c). In this way, while obtaining excess amounts of MDI polymer, a mixture of diisocyanates and polyisocyanates containing 50 to 80 wt.% of 4,4'-diphenylmethane diisocyanate, 1 to 12 wt.% of 2,4'- and/or 2,2'-diphenylmethane diisocyanate taken together, and 10 to 45 wt.% of trifunctional and higher-functional polyisocyanates, based on the weight of the mixture of diisocyanates and polyisocyanates, can be obtained from a mixture poorer in 4,4'-MDI, it being possible to choose the 4,4'-MDI content freely within wide limits. This embodiment can be particularly advantageous if the production plant on the first production site makes only a fraction of the diisocyanates and polyisocyanates produced available for the preparation of 4,4'-MDI on a second production site and also markets, e.g., MDI polymer as a finished product.

In a further embodiment of the present invention, as in the second, the process is first carried out up to the preparation of the partial distillate having a preferred composition of 85 to 95 wt.% of 4,4'-MDI, 5 to 15 wt.% of 2,4'-MDI and max. 1 wt.% of 2,2'-MDI, and the bottom product (MDI polymer) having a preferred composition of 35 to 45 wt.% of 4,4'-MDI, 2 to 7 wt.% of 2,4'-MDI and less than 1 wt.% of 2,2'-MDI, together with 50 to 60 wt.% of trifunctional and higher-functional polyisocyanates. The partial distillate is then worked up into technically pure 4,4'-MDI (with a maximum of 3 wt.% of 2,4'-MDI) and a secondary stream by multistage distillation (e.g., according to EP-A-79516) or crystallization or by a combination of distillation and crystallization steps. The pure 4,4'-MDI is then mixed, in a weight ratio preferably of 1:1 to 1:3 (pure 4,4'-MDI to MDI polymer), with MDI polymer having a preferred composition of 35 to 45 wt.% of 4,4'-MDI, 2 to 7 wt.% of 2,4'-MDI and less than 1 wt.% of 2,2'-MDI, together with 50 to 60 wt.% of trifunctional and higher-functional polyisocyanates, to give the transportable mixture of diisocyanates and polyisocyanates transferred in step c), containing 50 to 80 wt.% of 4,4'-diphenylmethane diisocyanate, 1 to 12 wt.% of 2,4'- and/or 2,2'-diphenylmethane diisocyanate taken together, and 10 to 45 wt.% of trifunctional and higher-functional polyisocyanates, based on the weight of the mixture of diisocyanates and polyisocyanates. To obtain high contents of 2,4'-MDI and/or 2,2'-MDI of up to 12 wt.%, it is also possible to prepare a bottom product (MDI polymer) with higher contents of 2,4'-MDI and/or 2,2'-MDI. An example of a suitable composition for the bottom product (MDI polymer) is 35 to 45 wt.% of 4,4'-MDI, 2 to 15 wt.% of 2,4'-MDI and less than 1 wt.% of 2,2'-MDI, together with 50 to 60 wt.% of trifunctional and higher-functional polyisocyanates. This mixture is thus obtained by mixing two industrially prepared and commercially available products (e.g., those products sold under the names Desmodur 44 M and Desmodur^{®} 44V20 which are commercially available from Bayer MaterialScience AG). If the quality of the MDI polymer is appropriate, this MDI mixture enables a pure 4,4'-MDI to be recovered, e.g. by a simple flash distillation. Despite the high expense on the first production site, this embodiment can be economically advantageous if the second production site is small and hence capable of producing 4,4'-MDI at minimal technical expense.

The mixtures of diisocyanates and polyisocyanates obtained in the above-described three embodiments of the present invention are suitable for bulk transportation over large distances, e.g. by ship, even with transportation times of several weeks.

In step c) the mixtures of diisocyanates and polyisocyanates containing 50 to 80 wt.% of 4,4'-diphenylmethane diisocyanate, 1 to 12 wt.% of 2,4'- and/or 2,2'-diphenylmethane diisocyanate taken together, and 10 to 45 wt.% of trifunctional and higher-functional polyisocyanates, based on the weight of the mixture of diisocyanates and polyisocyanates, are then transferred to transport containers, for example vats, liquid transport containers, bulk containers or tankers, and transported to the second production site remote from the first production site, for example, several thousand kilometers away. This can give rise to transportation times of several weeks.

The mixtures of diisocyanates and polyisocyanate transferred and transported in step c) conventionally have a crystallization point below 20°C. They can optionally contain a maximum of 1 wt.%, preferably of less than 0.1 wt.%, based on the weight of the mixture, of organic, homogeneously dissolved solvents or diluents. Particularly suitable solvents or diluents are chlorinated aromatic compounds such as chlorobenzene or dichlorobenzene.

The processing of the mixture of diisocyanates and polyisocyanates containing 50 to 80 wt.% of 4,4'-diphenylmethane diisocyanate, 1 to 12 wt.% of 2,4'- and/or 2,2'-diphenylmethane diisocyanate taken together, and 10 to 45 wt.% of trifunctional and higher-functional polyisocyanates, based on the weight of the mixture of diisocyanates and polyisocyanates, on the second production site, which is conventionally smaller than the first production site in respect of the capacity to prepare diisocyanates and polyisocyanates of the diphenylmethane series, takes place in known manner (See, e.g., DE-A-1938384, DE-A-2631168, EP-A-79516, EP-A-1475367). The first process step at the second site is typically a separation of a fraction of the diphenylmethane diisocyanate from the higher homologues by distillation or crystallization. Commercially conventional MDI polymers of different viscosities are obtained as the bottom product. The preferred composition of the MDI polymer is 35 to 45 wt.% of 4,4'-MDI, 2 to 7 wt.% of 2,4'-MDI and less than 1 wt.% of 2,2'-MDI, together with 50 to 60 wt.% of trifunctional and higher-functional polyisocyanates. Depending on the purity of the mixture of diisocyanates and polyisocyanates supplied (especially in the third embodiment), the diphenylmethane diisocyanate separated off may already satisfy the quality requirements for 4,4'-MDI. If this is not the case, the diphenylmethane diisocyanate separated off is freed of the 2,2' and 2,4' isomers and other impurities, conventionally in several distillation or crystallization steps, to give pure 4,4'-MDI containing at least 97 wt.% of 4,4'-diphenylmethane diisocyanate and a maximum of 3 wt.% of 2,4'-diphenylmethane diisocyanate. The secondary streams separated off can be used individually, admixed to the concomitantly produced MDI polymer or else optionally disposed of in small fractions.

The Examples which follow are intended to illustrate the invention in greater detail without however limiting its scope.

### EXAMPLES

### Example 1

### a) Preparation of the polyamine mixture:

In a stirred vessel, 2600 g of aniline were intimately mixed at 25°C with 1000 g of formalin (30 wt.% aqueous solution), with stirring until the mixture warmed up to 60°C. The stirrer was stopped and the upper, aqueous phase was separated off. 68 g of 30 wt.% aqueous hydrochloric acid were then admixed, with renewed stirring and cooling. The temperature was kept at 45°C. After stirring for a further 15 min at this temperature, the cooling was replaced by heating and the mixture was heated uniformly to 140°C over 120 min under 5 bar pressure and then kept at this temperature for 5 min.

The mixture was then cooled to 100°C, let down to normal pressure and neutralized by adding 54 g of 50 wt.% aqueous sodium hydroxide solution, with stirring. After the stirrer had been stopped, the phases were left to settle and the lower, aqueous phase was siphoned off. Excess aniline with residual water was then distilled off, initially under normal pressure, and the aniline residues were removed by distilling the resulting polyamine mixture at 100 mbar and 250°C.

This yielded 1900 g of a polyamine mixture having the following composition:
4,4'-MDA: 60.1 wt.%
2,4'-MDA: 6.0 wt.%
2,2'-MDA: 0.2 wt.%
higher-molecular polyamines: 33.7 wt.%

### b) Preparation of the polyisocyanate mixture:

In another stirred reactor, the 1900 g of polyamine mixture were dissolved in 5700 g of chlorobenzene. In a second vessel, a 33 wt.% phosgene solution was prepared by dissolving 3800 g of phosgene in 7600 g of chlorobenzene, with cooling to 0°C, and mixing the amine and phosgene solutions with vigorous stirring. The solid suspension formed was then heated slowly and the hydrogen chloride gas produced was withdrawn in appropriate manner. This gave a homogeneous solution of the polyisocyanate. The solvent was then separated off by distillation to give 2370 g of a polyisocyanate mixture having the following composition:
4,4'-MDI: 59.3 wt.%
2,4'-MDI: 5.5 wt.%
2,2'-MDI: 0.2 wt.%
higher-molecular polyisocyanates: 35 wt.%

This mixture had a crystallization point below 20°C and was suitable for transportation in large barrels and tankers.

### c) Preparation of 4,4'-MDI:

In a pot still the polyisocyanate mixture from b) was distilled at 10 mbar pressure and 215°C bottom temperature until 950 g of distillate had been obtained. 1420 g of bottom product remained.

The distillate was worked up in a baffle column corresponding to that described in EP-A-1475367, 59 g/h of distillate were introduced into the column in the region of the baffle. A bottom stream of 51 g/h with a 4,4'-MDI content of 98 wt.% and a 2,4'-MDI content of 2% was withdrawn from the baffle column. A top stream of 0.7 g/h having a composition of 25 wt.% of 2,2'-MDI, 73 wt.% of 2,4'-MDI and 2 wt.% of 4,4'-MDI, and a side stream of 6.1 g/h having a composition of 42.6 wt.% of 2,4'-MDI and 57.4 wt.% of 4,4'-MDI, were also withdrawn; these two secondary streams could be admixed to the bottom product again.

Fabric packings with a specific surface area of 500 m²/m³ were used as material exchange elements in the baffle column. The rectification zone and the stripping zone each had 8 separation stages and the pre-fractionation zone and the main fractionation zone each had 12 separation stages at the top and bottom, i.e. above and below the point of introduction of the feed stream into the pre-fractionation zone or above and below the point of withdrawal of the side stream from the main fractionation zone. The top pressure was 6 mbar. The reflux was 90:1 at the distillate withdrawal point and 2.6:1 at the side stream withdrawal point.

### Example 2

### a) Preparation of the polyamine mixture:

In a stirred vessel, 1800 g of aniline were intimately mixed at 30°C with 1000 g of formalin (30 wt.% aqueous solution), with stirring. The mixture was warmed up to 80°C. The stirrer was stopped and the upper, aqueous phase was separated off. 23 g of 30 wt.% aqueous hydrochloric acid were then admixed, with renewed stirring and cooling. The temperature was kept at 45°C. After stirring for a further 15 min at this temperature, the cooling was replaced by heating and the mixture was heated uniformly to 140°C over 150 min under 5 bar pressure and then kept at this temperature for 20 min.

The mixture was then cooled to 100°C, let down to normal pressure and neutralized by adding 18 g of 50 wt.% aqueous sodium hydroxide solution, with stirring. After the stirrer had been stopped, the phases were left to settle and the lower, aqueous phase was siphoned off. Excess aniline with residual water was then distilled off, initially under normal pressure, and the aniline residues were removed by distilling the resulting polyamine mixture at 100 mbar and 250°C. This gave 1880 g of a polyamine mixture having the following composition:
4,4'-MDA: 44.5 wt.%
2,4'-MDA: 7.3 wt.%
2,2'-MDA: 0.5 wt.%
higher-molecular polyamines: 47.7 wt.%

### b) Preparation of the polyisocyanate mixture:

The polyamine mixture was reacted with phosgene in chlorobenzene in the same manner as described in Example 1 to give the polyisocyanate mixture.

This gave 2330 g of a polyisocyanate mixture having the following composition:
4,4'-MDI: 44.1 wt.%
2,4'-MDI: 7.2 wt.%
2,2'-MDI: 0.5 wt.%
higher-molecular polyisocyanates: 48.2 wt.%

This first polyisocyanate mixture was distilled in a pot still at 10 mbar pressure and 215°C bottom temperature until 280 g of distillate had been obtained. This distillate was back-mixed with 420 g of the remaining bottom product. This gave 700 g of a second polyisocyanate mixture having the following composition:
4,4'-MDI: 57.4 wt.%
2,4'-MDI: 9.0 wt.%
2,2'-MDI: 0.5 wt.%
higher-molecular polyisocyanates: 33.1 wt.%

This mixture had a crystallization point below 20°C and was suitable for transportation in large barrels and tankers.

### c) Preparation of 4,4'-MDI:

In a pot still, the second polyisocyanate mixture from b) was distilled at 10 mbar pressure and 215°C bottom temperature until 280 g of distillate had been obtained. 420 g of bottom product remained.

The distillate was worked up in a baffle column corresponding to that described in EP-A-1475367 as in Example 1. 59 g/h of distillate were introduced into the column in the region of the baffle. A bottom stream of 46.9 g/h with a 4,4'-MDI content of 98 wt.% and a 2,4'-MDI content of 2 wt.% was withdrawn from the baffle column. A top stream of 1.2 g/h having a composition of 25 wt.% of 2,2'-MDI, 73 wt.% of 2,4'-MDI and 2 wt.% of 4,4'-MDI, and a side stream of 10.7 g/h having a composition of 54.9 wt.% of 2,4'-MDI and 44.3 wt.% of 4,4'-MDI, were also withdrawn. These two secondary streams could be admixed to the bottom product again.

## Claims

1. A process for the preparation of 4,4'-diphenylmethane diisocyanate comprising:
a) reacting aniline and formaldehyde in the presence of an acid catalyst to produce diamines and polyamines of the diphenylmethane series at a first site,
b) reacting the diamines and polyamines with phosgene to produce corresponding diisocyanates and polyisocyanates of the diphenylmethane series at the first site,
c) optionally, separating the diisocyanates and polyisocyanates by distillation to give a mixture comprising: 50 to 80 wt.% of 4,4'-diphenylmethane diisocyanate, 1 to 12 wt.% of 2,4'- and/or 2,2'-diphenylmethane diisocyanate taken together, and 10 to 45 wt.% of trifunctional and higher-functional polyisocyanates, based on total weight of the mixture of diisocyanates and polyisocyanates,
d) transferring the mixture of diisocyanates and polyisocyanates to transport containers,
e) transporting the transport containers containing diisocyanates and polyisocyanates to a second site remote from the first site, and
f) separating the mixture of diisocyanates and polyisocyanates by distillation and/or crystallization to give a pure 4,4'-diphenylmethane diisocyanate comprising at least 97 wt.% of 4,4'-diphenylmethane diisocyanate and a maximum of 3 wt.% of 2,4'-diphenylmethane diisocyanate.

2. The process of Claim 1 in which a mixture comprising at least 98 wt.% of 4,4'-diphenylmethane diisocyanate, a maximum of 2 wt.% of 2,4'-diphenylmethane diisocyanate and a maximum of 0.1 wt.% of 2,2'-diphenylmethane diisocyanate is obtained in step f).

3. The process of Claim 1 in which a first fraction of the total amount of diisocyanate and polyisocyanate prepared in step b) by phosgenation is transferred to transport containers and transported to the second production site, optionally after distillative separation.

4. The process of Claim 3 in which a second fraction of the total amount of diisocyanate and polyisocyanate prepared in step b) is further processed at the first production site or at a third production site.

5. The process of Claim 1 in which a mixture comprising 55 to 75 wt.% of 4,4'-diphenylmethane diisocyanate, 4 to 10 wt.% of 2,4'- and/or 2,2'-diphenylmethane diisocyanate taken together, and 20 to 40 wt.% of trifunctional and higher-functional polyisocyanates, based on total weight of the mixture of diisocyanates and polyisocyanates is obtained in step c).

6. The process of Claim 1 in which a mixture comprising from 50 to 70 wt.% of 4,4'-diphenylmethane diisocyanate, from 5 to 9 wt.% of 2,4'- and/or 2,2'-diphenylmethane diisocyanate taken together, and from 30 to 40 wt.% of trifunctional and higher-functional polyisocyanates, based on total weight of the mixture of diisocyanates and polyisocyanates is obtained in step c).

7. The process of Claim 1 in which aniline and formaldehyde are reacted in a molar ratio of from 1.9:1 to 3:1 and aniline and hydrochloric acid are used in a molar ratio of from 10:1 to 2:1 in step a).

8. The process of Claim 7 in which the mixture of diamines and polyamines produced in step a) comprises from 50 to 80 wt.% of 4,4'-diphenylmethane diamine, from 1 to 12 wt.% of 2,4'- and 2,2'-diphenylmethane diamine taken together, and from 10 to 45 wt.% of trifunctional and higher-functional polyamines, based on total weight of the mixture of diamines and polyamines.

9. The process of Claim 1 in which the diisocyanates and polyisocyanates produced in step b) are separated by flash evaporation under vacuum into a partial distillate comprising from 85 to 95 wt.% of 4,4'-diphenylmethane diisocyanate, from 5 to 15 wt.% of 2,4'-diphenylmethane diisocyanate and a maximum of 1 wt.% of 2,2'-diphenylmethane diisocyanate and a bottom product.

10. The process of Claim 9 in which the bottom product comprises from 35 to 45 wt.% of 4,4'-diphenylmethane diisocyanate, from 2 to 7 wt.% of 2,4'-diphenylmethane diisocyanate, less than 1 wt.% of 2,2'-diphenylmethane diisocyanate, and from 50 to 60 wt.% of trifunctional and higher-functional polyisocyanates is obtained from the flash separation.

11. The process of Claim 9 in which the partial distillate is mixed with the bottom product in a proportion such that a mixture comprising from 50 to 80 wt.% of 4,4'-diphenylmethane diisocyanate, from 1 to 12 wt.% of 2,4'-and/or 2,2'-diphenylmethane diisocyanate taken together, and from 10 to 45 wt.% of trifunctional and higher-functional polyisocyanates, based on total weight of the mixture of diisocyanates and polyisocyanates is obtained.

12. The process of Claim 9 in which the bottom product comprises from 35 to 45 wt.% of 4,4'-diphenylmethane diisocyanate, from 2 to 15 wt.% of 2,4'-diphenylmethane diisocyanate, less than 1 wt.% of 2,2'-diphenylmethane diisocyanate, and from 50 to 60 wt.% of trifunctional and higher-functional polyisocyanates.

13. The process of Claim 12 in which the pure 4,4'-diphenylmethane diisocyanate from step f) is mixed with the bottom product in a proportion such that a mixture of diisocyanates and polyisocyanates comprising from 50 to 80 wt.% of 4,4'-diphenylmethane diisocyanate, from 1 to 12 wt.% of 2,4'- and/or 2,2'-diphenylmethane diisocyanate taken together, and from 10 to 45 wt.% of trifunctional and higher-functional polyisocyanates, based on total weight of the mixture of diisocyanates and polyisocyanates is obtained.

## Patentansprüche

1. Verfahren zur Herstellung von 4,4'-Diphenylmethandiisocyanat, umfassend:
a) die Reaktion von Anilin mit Formaldehyd in der Gegenwart eines Säure-Katalysators zur Herstellung von Di- und Polyaminen der Diphenylmethan-Reihe an einer ersten Stelle,
b) die Reaktion der Di- und Polyamine mit Phosgen zur Herstellung der entsprechenden Di- und Polyisocyanate der Diphenylmethan-Reihe an der ersten Stelle,
c) gegebenenfalls die Abtrennung der Di- und Polyisocyanate durch Destillation, um eine Mischung zu ergeben, die umfasst: 50 bis 80 Gew.-% 4,4'-Diphenylmethandiisocyanat, 1 bis 12 Gew.-% von zusammen 2,4'- und/oder 2,2'-Diphenylmethandiisocyanat sowie 10 bis 45 Gew.-% tri- und höher-funktionelle Polyisocyanate, bezogen auf das Gesamtgewicht der Mischung aus den Di- und Polyisocyanaten,
d) die Überführung der Mischung aus den Di- und Polyisocyanaten in Transportbehälter,
e) den Transport der Transportbehälter, die die Di- und Polyisocyanate enthalten, zu einer zweiten Stelle, die entfernt von der ersten Stelle vorliegt, und
f) die Auftrennung der Mischung aus den Di- und Polyisocyanaten durch Destillation und/oder Kristallisation, um ein reines 4,4'-Diphenylmethandiisocyanat zu ergeben, das mindestens 97 Gew.-% 4,4'-Diphenylmethandiisocyanat und maximal 3 Gew.-% 2,4'-Diphenylmethandiisocyanat umfasst.

2. Verfahren gemäß Anspruch 1, wobei eine Mischung in der Stufe f) erhalten wird, die mindestens 98 Gew.-% 4,4'-Diphenylmethandiisocyanat, maximal 2 Gew.-% 2,4'-Diphenylmethandiisocyanat und maximal 0,1 Gew.-% 2,2'-Diphenylmethandiisocyanat umfasst.

3. Verfahren gemäß Anspruch 1, wobei eine erste Fraktion der Gesamtmenge der durch die Phosgenierung in Stufe b) hergestellten Di- und Polyisocyanate in Transportbehälter überführt und zur zweiten Produktionsstelle, gegebenenfalls nach destillativer Auftrennung, transportiert wird.

4. Verfahren gemäß Anspruch 3, wobei die in Stufe b) hergestellte zweite Fraktion der Gesamtmenge der Di- und Polyisocyanate an der ersten Produktionsstelle oder an einer dritten Produktionsstelle weiterverarbeitet wird.

5. Verfahren gemäß Anspruch 1, wobei eine Mischung in Stufe c) erhalten wird, die 55 bis 75 Gew.-% 4,4'-Diphenylmethandiisocyanat, 4 bis 10 Gew.-% von zusammen 2,4'- und/oder 2,2'-Diphenylmethandiisocyanat und 20 bis 40 Gew.-% tri- und höher-funktionelle Polyisocyanate umfasst, bezogen auf das Gesamtgewicht der Mischung aus den Di- und Polyisocyanaten.

6. Verfahren gemäß Anspruch 1, wobei eine Mischung in Stufe c) erhalten wird, die 50 bis 70 Gew.-% 4,4'-Diphenylmethandiisocyanat, 5 bis 9 Gew.-% von zusammen 2,4'- und/oder 2,2'-Diphenylmethandiisocyanat und 30 bis 40 Gew.-% tri- und höher-funktionelle Polyisocyanate umfasst, bezogen auf das Gesamtgewicht der Mischung aus den Di- und Polyisocyanaten.

7. Verfahren gemäß Anspruch 1, wobei das Anilin und der Formaldehyd in einem Molverhältnis von 1,9:1 bis 3:1 zur Reaktion gebracht und das Anilin und Salzsäure in einem Molverhältnis von 10:1 bis 2:1 in der Stufe a) verwendet werden.

8. Verfahren gemäß Anspruch 7, wobei die in Stufe a) erzeugte Mischung der Di- und Polyamine 50 bis 80 Gew.-% 4,4'-Diphenylmethandiamin, 1 bis 12 Gew.-% von zusammen 2,4'- und 2,2'-Diphenylmethandiamin und 10 bis 45 Gew.-% tri- und höher-funktionelle Polyamine umfasst, bezogen auf das Gesamtgewicht der Mischung aus den Di- und Polyaminen.

9. Verfahren gemäß Anspruch 1, wobei die in Stufe b) erzeugten Di- und Polyisocyanate durch eine Blitz-Verdampfung unter Vakuum in ein Teildestillat aufgetrennt werden, das 85 bis 95 Gew.-% 4,4'-Diphenylmethandiisocyanat, 5 bis 15.Gew.-% 2,4-Diphenylmethandiisocyanat und maximal 1 Gew.-% 2,2'-Diphenylmethandiisocyanat und ein Sumpfprodukt umfasst.

10. Verfahren gemäß Anspruch 9, wobei das Sumpfprodukt, das 35 bis 45 Gew.-% 4,4'-Diphenylmethandiisocyanat, 2 bis 7 Gew.-% 2,4'-Diphenylmethandiisocyanat, weniger als 1 Gew.-% 2,2'-Diphenylmethandiisocyanat und 50 bis 60 Gew.-% tri- und höher-funktionelle Polyisocyanate umfasst, aus der Blitz-Auftrennung erhalten wird.

11. Verfahren gemäß Anspruch 9, wobei das Teildestillat mit dem Sumpfprodukt in einem solchen Mengenanteil vermischt wird, dass eine Mischung erhalten wird, die 50 bis 80 Gew.-% 4,4'-Diphenylmethandiisocyanat, 1 bis 12 Gew.-% von zusammen 2,4'- und/oder 2,2'-Diphenylmethandiisocyanat und 10 bis 45 Gew.-% tri- und höher-funktionelle Polyisocyanate umfasst, bezogen auf das Gesamtgewicht der Mischung aus den Di- und Polyisocyanaten.

12. Verfahren gemäß Anspruch 9, wobei das Sumpfprodukt 35 bis 45 Gew.-% 4,4'-Diphenylmethandiisocyanat, 2 bis 15 Gew.-% 2,4'-Diphenylmethandiisocyanat, weniger als 1 Gew.-% 2,2'-Diphenylmethandiisocyanat und 50 bis 60 Gew.-% tri- und höher-funktionelle Polyisocyanate umfasst.

13. Verfahren gemäß Anspruch 12, wobei das reine 4,4'-Diphenylmethandiisocyanat aus der Stufe f) mit dem Sumpfprodukt in einem solchen Mengenverhältnis vermischt wird, dass eine Mischung der Di- und Polyisocyanate erhalten wird, die 50 bis 80 Gew.-% 4,4'-Diphenylmethandiisocyanat, 1 bis 12 Gew.-% von zusammen 2,4'- und/oder 2,2'-Diphenylmethandiisocyanat und 10 bis 45 Gew.-% der tri- und höher-funktionellen Polyisocyanate umfasst, bezogen auf das Gesamtgewicht der Mischung aus den Di- und Polyisocyanaten.

## Revendications

1.- Procédé pour la préparation de 4,4'-diphénylméthane diisocyanate comprenant :
a) la réaction d'aniline et de formaldéhyde en présence d'un catalyseur acide pour produire des diamines et des polyamines de la série du diphénylméthane, à un premier site de production, et
b) la réaction des diamines et des polyamines avec du phosgène pour donner les diisocyanates et les polyisocyanates correspondants de la série du diphénylméthane au premier site de production,
c) facultativement, la séparation des diisocyanates et des polyisocyanates par distillation pour donner un mélange comprenant : 50 à 80% en poids de 4,4'-diphénylméthane diisocyanate, 1 à 12% en poids de 2,4'- et/ou 2,2'-diphénylméthane diisocyanate pris conjointement, et 10 à 45% en poids de polyisocyanates trifonctionnels et de fonctionnalité supérieure, sur base du poids total du mélange des diisocyanates et des polyisocyanates,
d) le transfert du mélange de diisocyanates et de polyisocyanates aux conteneurs de transport
e) le transport des conteneurs de transport contenant les diisocyanates et les polyisocyanates à un second site de production éloigné du premier, et
f) la séparation du mélange de diisocyanates et de polyisocyanates par distillation et/ou cristallisation pour donner un 4,4'-diphénylméthane diisocyanate pur comprenant au moins 97% en poids de 4,4'-diphénylméthane diisocyanate et un maximum de 3% en poids de 2,4'-diphénylméthane diisocyanate.

2. Procédé selon la revendication 1 dans lequel un mélange comprenant au moins 98% en poids de 4,4'-diphénylméthane diisocyanate, un maximum de 2% en poids de 2,4'-diphénylméthane diisocyanate et un maximum de 0,1% en poids de 2,2'-diphénylméthane diisocyanate est obtenu dans l'étape f).

3. Procédé selon la revendication 1 dans lequel une première fraction de la quantité totale de diisocyanate et de polyisocyanate préparée dans l'étape b) par phosgénation est transférée dans des conteneurs de transport et transportée au second site de production, facultativement après séparation par distillation.

4. Procédé selon la revendication 3 dans lequel une seconde fraction de la quantité totale de diisocyanate et de polyisocyanate préparée dans l'étape b) est en plus traitée au premier site de production ou à un troisième site de production.

5. Procédé selon la revendication 1 dans lequel un mélange comprenant au moins 55 à 75% en poids de 4,4'-diphénylméthane diisocyanate, 4 à 10% en poids de 2,4'- et/ou 2,2'-diphénylméthane diisocyanate pris conjointement, et 20 à 40% en poids de polyisocyanates trifonctionnels et de fonctionnalité supérieure, sur base du poids total du mélange de diisocyanates et de polyisocyanates est obtenu dans l'étape c).

6. Procédé selon la revendication 1 dans lequel un mélange comprenant de 50 à 70% en poids de 4,4'-diphénylméthane diisocyanate, de 5 à 9% en poids de 2,4'- et/ou 2,2'-diphénylméthane diisocyanate pris conjointement, et de 30 à 40% en poids de polyisocyanates trifonctionnels et de fonctionnalité supérieure, sur base du poids total du mélange de diisocyanates et de polyisocyanates est obtenu dans l'étape c).

7. Procédé selon là revendication 1 dans lequel l'aniline et le formaldéhyde sont mis à réagir dans un rapport molaire de 1:9 à 3:1 et l'aniline et l'acide chlorhydrique sont utilisés dans un rapport molaire de 10:1 à 2:1 dans l'étape a).

8. Procédé selon la revendication 7 dans lequel le mélange de diamines et de polyamines produit dans l'étape a) comprend de 50 à 80% en poids de 4,4'-diphénylméthane diamine, de 1 à 12% en poids de 2,4'- et/ou 2,2'-diphénylméthane diamine prises conjointement, et de 10 à 45% en poids de polyamines trifonctionnelles et de fonctionnalité supérieure, sur base du poids total du mélange de diamines et de polyamines.

9. Procédé selon la revendication 1 dans lequel les diisocyanates et les polyisocyanates produits dans l'étape b) sont séparés par évaporation éclair sous vide en un distillat partiel comprenant de 85 à 95% en poids de 4,4'-diphénylméthane diisocyanate, de 5 à 15% en poids de 2,4'-diphénylméthane diisocyanate et un maximum de 1% en poids de 2,2'-diphénylméthane diisocyanate et un produit de fond.

10. Procédé selon la revendication 9 dans lequel le produit de fond comprend de 35 à 45% en poids de 4,4'-diphénylméthane diisocyanate, de 2 à 7% en poids de 2,4'-diphénylméthane diisocyanate, moins de 1% en poids de 2,2'-diphénylméthane diisocyanate et de 50 à 60% en poids de polyisocyanates trifonctionnels et de fonctionnalité supérieure, est obtenu par séparation éclair.

11. Procédé selon la revendication 9 dans lequel le distillat partiel est mélangé avec le produit de fond dans une proportion telle qu'un mélange comprenant de 50 à 80% en poids de 4,4'-diphénylméthane diisocyanate, de 1 à 12% en poids de 2,4'- et/ou 2,2'-diphénylméthane diisocyanate pris conjointement, et de 10 à 45% en poids de polyisocyanates trifonctionnels et de fonctionnalité supérieure, sur base du poids total du mélange de diisocyanates et de polyisocyanates, est obtenu.

12. Procédé selon la revendication 9 dans lequel le produit de fond comprend de 35 à 45% en poids de 4,4'-diphénylméthane diisocyanate, de 2 à 15% en poids de 2,4'-diphénylméthane diisocyanate, moins de 1% en poids de 2,2'-diphénylméthane diisocyanate et de 50 à 60% en poids de polyisocyanates trifonctionnels et de fonctionnalité supérieure.

13. Procédé selon la revendication 12 dans lequel le 4,4'-diphénylméthane diisocyanate pur de l'étape f) est mélangé avec le produit de fond dans une proportion telle qu'un mélange de diisocyanates et de polyisocyanates comprenant de 50 à 80% en poids de 4,4'-diphénylméthane diisocyanate, de 1 à 12% en poids de 2,4'- et/ou de 2,2'-diphénylméthane diisocyanate pris conjointement, et de 10 à 45% en poids de polyisocyanates trifonctionnels et de fonctionnalité supérieure, est obtenu.
